# EUROPÄISCHE PATENTANMELDUNG

(11) **EP 1 059 117 A2**
(43) Veröffentlichungstag der Anmeldung: **13.12.2000**
(21) Anmeldenummer: 00117777.3
(22) Anmeldetag: 17.09.1991
(51) Int. Cl.: B01J 20/26, A61L 15/22, A61L 15/60

(54) **Verfahren zur Herstellung von Absorptionsmaterial auf Polymerbasis mit verbesserter Abbaubarkeit und Absorption von Wasser, wässrigen Lösungen und Körperflüssigkeiten und die Verwendung in Hygieneartikeln und zur Bodenverbesserung**

(30) Priorität: 19.09.1990 DE 4029593
(62) Teilanmeldung aus: 91115708.9
(71) Anmelder: STOCKHAUSEN GmbH & Co. KG, 47805 Krefeld (DE)
(72) Erfinder: Chmelir, Miroslav, Dr., 47803 Krefeld (DE)
(74) Vertreter: Kutzenberger, Helga, Dr.

(57) **Zusammenfassung**

Die Erfindung betrifft ein Verfahren zur Herstellung von Absorptionsmaterial für Wasser, wässrige Lösungen und Körperflüssigkeiten aus wenigstens zwei Komponenten A und B, wobei die Komponente A wenigstens ein wasserquellbares synthetisches Polymeres oder Copolymeres und die Komponente B wenigstens eine natürliche oder synthetische, polymere Verbindung ist, die bei normaler Temperatur als rieselfähiges Pulver vorliegt und in Wasser nur begrenzt löslich oder unlöslich ist. Die Erfindung ist dadurch gekennzeichnet, daß die Komponente B in trockener oder in teilweise gequollener Form der Komponente A während ihres Herstellungsprozesses zugesetzt wird, nachdem ein Polymerumsatz von mindestens 30 %, bevorzugt mindestens 60 %, erreicht worden ist, mit dem Polymergel der Komponente A vermischt und anschließend getrocknet wird. Die Erfindung betrifft ferner die Verwendung des Absorptionsmaterials zur Aufnahme und/oder Zurückhaltung von Wasser und/oder wässrigen Lösungen, insbesondere von wässrigen Körperflüssigkeiten wie Urin oder Blut, in absorbierenden Wegwerferzeugnissen für hygienische, chirurgische und andere medizinische Zwecke wie Babywindeln, Tampons und Damenbinden, zur Aufnahme und/oder Zurückhaltung von Wasser und/oder wässrigen Lösungen und zur nachfolgenden gesteuerten Abgabe von Wasser und/oder der in wässrigem Medium gelösten Substanzen an andere Körper sowie zur Trocknung von Gasen und/oder Flüssigkeiten, bevorzugt von organischen, mit Wasser nicht mischbaren Flüssigkeiten und Lösungsmitteln.

## Beschreibung

Die Erfindung betrifft ein Verfahren zur Herstellung von Absorptionsmaterialien, die aus einer Kombination von synthetischen Polymeren und bevorzugt Naturpolymeren, wie z.B. Polysacchariden, bestehen, die Wasser und wässrige Flüssigkeiten schnell aufnehmen und die z.B. für absorbierende Wegwerferzeugnisse in Hygieneartikeln, wie Windeln und Damenbinden, und für andere medizinische Zwecke oder auch als wasserspeichernde Bodenverbesserungsmittel verwendet werden.

Absorptionsmittel mit hohem Absorptionsvermögen für Wasser und Körperflüssigkeiten sind bekannt. Zu den vollsynthetischen Absorptionsmitteln gehören vernetzte Polymere und Copolymere auf Acryl- oder Methacrylsäurebasis (DE-OS 24 29 236, DE-OS 26 14 662, US-PS 4 018 951, US-PS 3 926 891, US-PS 4 066 583, US-PS 4 062 817, DE-OS 27 12 043, DE-OS 26 53 135, DE-OS 26 50 377, DE-OS 28 13 634), Maleinsäurederivate (nach US-PS 4 041 228) oder Acrylamidopropansulfonsäurecopolymerisate (nach DE-PS 31 24 008). Diese bekannten synthetischen Absorptionsmittel sind praktisch wasserunlöslich, absorbleren im Gleichgewicht das Vielfache ihres Gewichts an Wasser, Urin oder anderen wässrigen Lösungen, sind jedoch relativ beständig gegen biologische Abbaubarkeit.

Weitere Produkte wurden auf Stärkebasis, wie z.B. Stärke-Acrylnitril-Pfropfpolymerisate (nach US-PS 3 997 484, US-PS 3 661 815, US-PS 4 155 888, US-PS 3 935 099), gelatinisierte Stärkederivate (nach DE-OS 27 02 781) oder auf Cellulosebasis, wie
Derivate von Alkyl- oder Hydroxyalkylcellulose (nach JP-PS 77/125 481), Carboxymethylcellulose (nach BE-PS 862 130, GB-PS 1 159 949) und auf Polysaccharidbasis (nach DE-OS 26 50 377) hergestellt. Diese Produkte, wie die mit Acrylnitril- oder Acrylsäure gepfropften Stärkepolymerisate gehören, zwar zu den abbaubaren Produkten, jedoch ist ihre Herstellung sehr aufwendig und die Menge des Naturprodukts im Endprodukt ist durch die hohe Viskosität des Reaktionsmediums, wie dies z.B. bei einer Monomerlösung mit gelöster Stärke der Fall ist, stark begrenzt. Beispielsweise enthält die Monomerenlösung nach EP 0 372 981 Beispiel 11 nur ca. 4 Gew% gelöste Stärke, bezogen auf den gesamten Ansatz.

Stärkeprodukte, Dextrin oder Mehl werden gemäß DE-PS 21 43 549 auch zur Bepuderung der Gallert-Granulate von Polyacrylamidpolymerisaten benutzt, um lagerfähige, nicht zusammenbackende, formstabile Gallert-Granulate mit hohem Wassergehalt zu erhalten, deren Verwendung als wasserlösliche Flockungs- und Sedimentationshilfsmittel vorgeschlagen wird.

In diesem Fall ist die Stärke nur auf der Oberfläche der wasserhaltigen Polymergelgranulate eines wasserlöslichen Produkts verteilt. Beim ersten Kontakt mit Wasser wird die Stärke von der Oberfläche des bepuderten Polymergels weitgehend abgespült. Eine Einbindung der Stärke in den Polyacrylamidkörper ist hier nicht vorhanden.

So ist ein Verfahren, das Naturprodukt (im weiteren als Komponente B bezeichnet) direkt zu der Monomerlösung der Komponente A (synthetisches Polymerisat) zuzusetzen, zu homogenisieren und danach die Polymerisation zu starten, nur bei niedrigen Gehalten von 2 bis höchstens 5 Gew.% an Komponente B in der Monomerlösung durchführbar, während aus technischen und wirtschaftlichen Gründen die Monomerkonzentration der Monomerlösung zwischen 20 - 30 Gew.% liegt. Solche Verfahren sind z.B. in den (DE-PS 26 12 846) GB 1490128 und EP-PS 0 189 163 beschrieben. Die Versuche, höhere Mengen als 5 Gew.% an Komponente B in der Monomerlösung zu lösen, führen zu hochviskosen bis gelartigen, unrührbaren Monomerlösungen, die keine homogene Vermischung mit den Katalysatorlösungen erlauben, so daß eine gleichmäßige, bis zu hohem Umsatz geführte Polymerisation unmöglich ist.

Auch inerte Füllmittel, wie Holzmehl, Zellstoffasern, Cellulose- oder Nylonflocken, Schlacke, Ton, Flugasche, Kohlenstaub und Düngemittel, werden gemäß DE-AS 22 64 027 als Zusätze bei der Vernetzung der wasserlöslichen Polymerisate verwendet. Die nichtvernetzten wasserlöslichen Polymerisate bzw. Copolymerisate werden als getrocknetes Pulver mit den inerten Füllmitteln vermischt, und nach einer Besprühung des Gemisches mit 15 - 85 Gew.% oder sogar noch mehr Wasser (in den meisten Beispielen werden 200 - 400 Gew.% Wasser, bezogen auf das Gemisch Polymerisat und Füllmittel, verwendet) wird das entstandene wasserhaltige Granulat durch ionisierende Bestrahlung (0,05 - 20,0 MeV) vernetzt. Nach der Trocknung entsteht ein z.T. wasserunlösliches polymeres Absorptionsmaterial, dessen Wasserabsorptionskapazität etwa das 5- bis 40fach des Trockengewichtes beträgt.

Aufgabe der Erfindung ist die Bereitstellung eines Verfahrens, das es ermöglicht, die bekannten, als Absorptionsmittel eingesetzten synthetischen Polymerisate mit hohem Aufnahmevermögen für Wasser und wässrige Flüssigkeiten wie Urin oder Blut durch Zusätze, die selbst ein vergleichsweise geringes Aufnahmevermögen für derartige Flüssigkeiten haben, einzubinden, ohne daß sich das Absorptionsvermögen des Verfahrensprodukts auch bei Einbindung von höheren Mengen an schlechtet absorbierenden Zusätzen nennenswert verschlechtert. Gleichzeitig wird in bestimmten Fällen noch die Abbaubarkeit verbessert.

Diese Aufgabe wird erfindungsgemäß durch die kennzeichnenden Merkmale von Patentanspruch 1 gelöst.

Die Herstellung der Komponente A erfolgt auf bekannte Weise. So kann die Herstellung der Komponente A als gequollenes Polymergel diskontinuierlich in einem Polymerisationsgefäß oder kontinuierlich auf einem endlosen Band erfolgen. Gemäß DE-PS 35 44 770 wird z.B. die Polymerisation in einer wässrigen Lösung, enthaltend das wasserlösliche Monomere und ggf. die Comonomeren, in einer Konzentration von 2,2 bis 8,3 Mole polymerisationsfähiger Doppelbindungen pro Kilo Monomerenlösung, insbesondere 3,5 bis 6,25 Mole (entsprechend 16 - 60 Gew.%, insbesondere 25 - 45 Gew.% Acrylsäure, wenn diese als Monomere verwendet wird) in einem Temperaturbereich von etwa - 10 bis 120°C durchgeführt.

Als Komponente A kommen in erster Linie die Polymerisate von Acrylsäure und Methacrylsäure, alleine als Homopolymerisat oder als Copolymerisat, ferner aber auch die Polymerisate von anderen wasserlöslichen Monomeren wie Acrylamid sowie polymerisationsfähige Säuren und ihre Salze, insbesondere Malein-, Fumar-, Itacon-, Vinylsulfon- oder 2-Acrylamido-2-methylpropansulfonsäure in Frage. Ferner können hydroxygruppenhaltige Ester polymerisationsfähiger Säuren, insbesondere die Hydroxyethyl- und Hydroxypropylester der Acryl- und der Methacrylsäure verwendet werden, ebenso aminogruppenhaltige und ammoniumgruppenhaltige Ester und Amide polymerisationsfähiger Säuren, wie die Dialkylaminoester, insbesondere die Dimethyl- und die Diethylaminoalkylester der Acryl- und der Methacrylsäure sowie die Trimethyl- und die Triethylammoniumalkylester und die entsprechenden Amide. Weiterhin werden in geringen Anteilen vernetzende Monomere, wie z. B. Monomere mit mehr als einer polymerisationsfähigen Gruppe im Molekül zusammen mit den vorstehenden Monomeren polymensiert.

Die vorstehenden Monomeren können allein zu vernetzten Homo- oder untereinander zu vernetzten Copolymerisaten polymerisiert werden.

In geringen Mengen können noch in Wasser schwerlösliche oder sogar wasserunlösliche Monomere wie (Meth)acrylnitril, Vinylpyridin und Vinylacetat copolymerisiert werden wie die Ester der Acryl- und/oder Methacrylsäure mit C₁-C₁₀-Alkoholen, Styrol und alkylierte Styrole. Im allgemeinen liegt der Anteil an den wasserlöslichen Monomeren bei 40 bis 100 Gew.%, bezogen auf die Gesamtheit der Monomeren. Der Anteil an den vernetzenden Monomeren liegt bei 0 bis 20 Gew.%, vorzugsweise bei 0,01 bis 2,0 Gew%, bezogen auf die Gesamtheit der Monomeren. Die wasserunlöslichen, hydrophoben Monomeren machen in der Regel 0 bis 40 Gew.% der Monomeren aus.

Als vernetzende Monomere seien bi- oder mehrfunktionelle Monomere, z.B. Amide, wie das Methylenbisacryl- bzw. -methacrylamid oder Ethylenbisacrylamid, ferner Ester der ungesättigten Mono-oder Polycarbonsäuren von Polyolen, wie Diacrylate oder Triacrylate, z.B. Butandiol- oder Ethylenglykoldiacrylat bzw. -methacrylat, Trimethylolpropantriacrylat, ferner Vinylmethacrylat und Allylverbindungen, wie Allyl(meth)acrylat, Triallylcyanurat, Maleinsäurediallylester, Polyallylester, Tetraallyloxiethan, Triallylamin, Tetraallylethylendiamin, Allylester der Phosphorsäure bzw. phosphorigen Säure, ferner vernetzungsfähige Monomere, wie die N-Methylolverbindungen von Amiden, wie Methacrylamid bzw. Acrylamid und die davon abgeleiteten Äther.

Die Polymerisation kann durch chemische Katalyse und/oder durch energiereiche Strahlung/Licht initiiert werden. Als geeignete Katalysatoren können z.B. Peroxiverbindungen wie Kaliumperoxidisulfat, Wasserstoffperoxid, organische Peroxide, wie Benzoylperoxid, tert. Butylhydroperoxid, tert. Butylperpivalat, Redox-Systeme, wie z.B. Kaliumperoxidisulfat-Natriumdisulfit, Wasserstoffperoxid-Hydroxylaminchlorid oder Azoinitiatoren, wie AIBN (2,2'-Azobis(isobutyronitril)) oder 2,2'-Azobis(2-amidinopropan)dihydrochlorid verwendet werden. Als Photoinitiatoren können z.B. Benzoin und seine Derivate, z.B. Benzoinether, wie Benzoin-Ethyl-Propyl-Ether, Benzil und seine Derivate, wie Benzilketale oder Acryldiazoniumsalze, Acetophenonderivate und andere alleine oder in Gemischen. Im allgemeinen liegt der Gehalt an Photoinitiatoren bei 0,002 bis 2,0 Gew.%, vorzugsweise 0,01 bis 0,2 Gew.%, bezogen auf die eingesetzten Monomeren. Der Gehalt an Katalysatoren liegt im allgemeinen bei 0,02 bis 5,0 Gew.%, vorzugsweise zwischen 0,20 bis 2,0 Gew.%, bezogen auf die Monomeren.

Als Komponente B werden erfindungsgemäß natürliche Polymerisate auf Polysaccharidbasis, wie modifizierte Cellulose und Cellulosederivate, z.B. Alkyl-, Hydroxyalkyl-, Carboxymethylcellulose, Gummiharze, z.B. Guar, Johannisbrotkernmehl, Tragacanthgummi, Gummi Arabicum, Pektin u.a., Stärke und Stärkederivate, wie z.B Mais-, Korn-, Kartoffelstärke, Amylose, Amylopektin, Dextrin, Dextran, modifizierte Stärke, Hydroxyethylstärke, kationische Stärke, Stärkepfropfpolymerisate u.a. verwendet.

Zusätzlich zu diesen Naturpolymeren als Komponente B können noch andere Materialien mit großer Oberfläche, wie z.B. Fasermaterial aus Naturfasern, bevorzugt Baumwolle-, Hanf-, Wolle- und Seidenfasern, weiterhin Fasermaterial aus Cellulosefasern, wie Viskose-, Acetat- und Triacetatfasern oder aus synthetischen Fasern auf der Basis von Polyester, Polyolefinen, Polyacrylnitril, Polyamid, Polyvinylalkohol, -acetat und -chlorid, Polyurethan, Polyvinylharnstoff und der Copolymerisate von diesen Polymeren, verwendet werden. Die Fasermaterialien können bevorzugt als Kurzschnittfaser von einer Länge von 0,1 bis 60 mm in die Komponente A eingearbeitet werden.

Weitere Materialien, wie Riechstoffe zur Parfümierung des Endproduktes, Desinfektionsmittel, antibakterielle Mittel, aber auch neutrale Füllstoffe, wie z.B. Holzmehl, Torf, gemahlene Walnußschalen, Kernobstschalen, chitinhaltiges Mehl, Sand, Erde für Pflanzenzucht oder andere Streckmittel, können auch als Zusatz verwendet werden. Letztlich kann auch die feingemahlene Komponente A getrocknet in Pulverform oder in teilgequollener Form allein als Komponente B zum Polymergel der Komponente A zugesetzt werden.

Das erfindungsgemäße Verfahren besteht darin, daß die Komponente B als Pulver in trockenem bzw. in leicht angefeuchtetem bis gequollenem Zustand während der Herstellung des synthetischen Polymeren (Komponente A) zu dem gequollen Polymergel der Komponente A zugesetzt wird. Vorteilhaft ist, wenn die Komponente B erst in der Endphase der Herstellung der Komponente A, d.h. erst nachdem ein Umsatz von mehr als 30 %, bevorzugt mehr als 60 %, und besonders bevorzugt mehr als 95 %, erreicht wird, zu dem gequollenen Polymergel der Komponente A zugesetzt, mit dem Polymergel vermischt und anschließend getrocknet wird.

Die Vermischung der beiden Komponenten kann in einer geeigneten Mischeinrichtung erfolgen. Vorteilhaft wird hierzu ein Mischer mit rotierendem Mischwerkzeug verwendet. Ein geeigneter Mischer besteht z.B. aus einem vertikal oder horizontal gelagerten Metallzylinder, dessen Mischwerkzeuge mit Leitschaufeln, die die Wände des Mischerzylinders gründlich abstreifen, ausgerüstet ist.

Für eine gleichmäßige Durchmischung der beiden Komponenten A und B kann auch ein Trogkneter mit Doppel-U-Querschnitt verwendet werden. Im Trog bewegt sich ein Knetwellenpaar gleich- oder gegensinnig, gleich oder verschieden schnell, wobei auch die Schaufelform je nach Mischgut gezielt gewählt werden kann. Zum kontinuierlichen Betrieb kann der Trogkneter mit einem Austragzylinder und einer Austragschnecke, die entweder gegenläufig zum Troginnern arbeitet und damit den Misch- und Knetvorgang verstärkt oder die in umgekehrter Richtung zur Austragung des Mischgutes geschaltet wird, ausgestattet werden.

Zu den weiteren, für einen kontinuierlichen Betrieb geeigneten Maschineneinrichtungen gehören: Einwellenmischer wie Einschneckenextruder, Ko-Kneter, Zweiwellenmischer mit gleichläufiger oder gegenläufiger Doppelschnecke, konische Cotruder-Schnecke, Zweiwellen**duchlauf**kneter und kontinuierliche Mehrwellengeräte, wie z.B ein Vierschneckenextruder.

Nach dem Vermischen wird die Polymergelmasse bei einer Temperatur im Bereich von 50 - 160 Gew.% getrocknet. Man erhält ein Endprodukt, in dem die Komponente B (z.B. ein Naturpolymer) im synthetischen Polymerisat so eingebunden ist, daß die mit Wasser extrahierbaren Anteile des Endprodukts deutlich niedriger, bevorzugt 30 Gew.% und insbesondere niedriger als 20 Gew.%, sind als bei einer physikalischen Mischung der Mischungskomponenten A und B.

Die Einbindung der Komponente B in das synthetische Polymerisat kann durch Zusatz von verschiedenen, oben schon erwähnten Katalysatoren in einer Menge von 0,01 bis 2,0 Gew.% und/oder oben bereits erwähnten mehrfunktionellen Monomeren in einer Menge von 0,05 bis 5,0 Gew.% zu der Komponente B oder Komponente A verstärkt werden. Die Katalysatoren oder mehrfunktionellen Verbindungen können z.B. als Lösung auf die Komponenten A oder B vor oder während der Abmischung aufgesprüht werden.

Als Bindemittel für die beiden Komponenten A und B kann man noch zusätzlich als Hilfsmittel zur Bindung der beiden Komponenten die nieder- oder hochmolekularen, wasserlöslichen oder wasserquellbaren Polymerisate auf synthetischer oder natürlicher Grundlage (z.B. Polysaccharide in gelöstem oder aufgequollenem Zustand) verwenden. Beispiele für wasserlösliche oder wasserquellbare Polymerisate auf synthetischer Basis sind die Polymeren oder Copolymeren auf der Basis von (Meth-)Acrylsäure oder (Meth-)Acrylsäurederivaten wie die Homo- oder Copolymere der Acryl-, Methacryl-, 2-Acrylamido-2-methylpropansulfonsäure, der Salze dieser Säuren, des Acryl- oder Methacrylamids untereinander oder mit Vinylpyrrolidon und/oder Vinylacetat sowie Polyvinylalkohol.

Es können aber auch nieder- oder hochmolekulare Polymerisate, die als Emulsionspolymerisate in einer wässrigen Dispersion in Form der durch Emulgator solubilisierten winzigen kugelförmigen Partikel vorhanden sind, verwendet werden, wobei beide Emulsionsformen 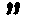 Öl-in-Wasser" (für wasserunlösliche Polymere) sowie 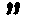 Wasser-in-Öl" (für wasserlösliche Polymere) möglich sind. Die Ölphase besteht bekanntlich meist aus mit Wasser nicht mischbaren organischen Lösungsmitteln wie aliphatischen oder aromatischen Kohlenwasserstoffen (z.B. Hexan, Weißöl).

Beispiele für Polymerisate, die Öl-in-Wasser-Emulsionen bilden konnen, sind Polymerisate von Butadien, Styrol, Isopren, Chloropren, Acrylnitril, Vinylacetat, Vinyl- und Vlnylidenchlorid, Alkylacrylate und -methacrylate und Copolymerisate von diesen Monomeren untereinander sowie auch mit Methylstyrol, Isobutylen oder Äthylen.

Als Beispiele für Polymerisate, die in Wasser-in-Öl-Emulsionen verwendet werden, sind die schon o.a. wasserlöslichen oder wasserquellbaren Polymerisate bzw. Copolymerisate auf Basis von (Meth)-Acrylsäurederivaten, die unvernetzt oder auch vernetzt sein können.

Die nach dem erfindungsgemäßen Verfahren aus Komponenten A und B hergestellten Endprodukte zeigen, wie in den nachfolgenden Beispielen dargestellt ist, ein verbessertes Aufnahmevernögen für synthetische Modellurinlösung (Beispiele 1 - 8), bei einer deutlich besseren Abbaubarkeit der Endprodukte (Beispiel 16).

Das Endprodukt besteht aus den Komponenten A und B in einen Gewichtsverhältnis 20 bis 98 Gew.%, vorzugsweise 40 bis 95 Gew.% der Komponente A, zu 2 bis 80 Gew.%, vorzugsweise 5 bis 60 Gew.%, der Komponente B.

### Prüfmethoden:

1) Zur Ermittlung der Aufnahmegeschwindigkeit wird die Aufnahme von Modellurin nach dem Demand-Absorbency-Test (DAT-Methode nach W. F. Schlauch, Vortrag Index 1978, Amsterdam) durchgeführt und die Aufnahmegeschwindigkeit nach 60 Sekunden sowie die Maximalaufnahme und Retention ermittelt. Das Meßgerät besteht aus einer Bürette, die mit der Modellurinlösung (2,0 % Harnstoff, 0,9 % NaCl, 0,1 % MgSO₄ und 0,06 % CaCl_{2,} aufgelöst in destilliertem Wasser) gefüllt ist, und einem Probetisch, der mit einer an die Meßbürette angeschlossenen Öffnung für den Modellurinlösungsaustritt versehen ist. Auf dem mit einem dünnen Vlies (10 x 13,5 cm) bedeckten Probetisch werden 0,5 g des erfindungsgemäßen Produktes, vermischt mit 5 mg Aerosil 200, in Form einer kreisrunden Fläche von 4,5 cm Durchmesser, zentrisch über dem Flüssigkeitsaustritt, gleichmaßig aufgestreut. Durch Schließen des Schlauches und leichte Druckgebung wird der Kontakt der Modellurinlösung mit dem Pulverprodukt hergestellt, so daß die Modellurinlösung durch das erfindungsgemäße Produkt absorbiert werden kann. Die absorbierte Menge der Modellurinlösung wird nach 20 - 30 Minuten als Maximalwert abgelesen. Anschließend wurde die Retention durch Belastung des gequollenen Geles mit einem Gewicht von 10 g/cm² ermittelt; die Zeit der Belastung betrug 5 Minuten. Die ermittelten Retentionswerte sind in den Beispielen tabellenweise zusammengefaßt.
2) Als weitere Methode zur Bestimmung der Flüssigkeitsaufnahmegeschwindigkeit wurde ein Teebeuteltest durchgeführt, wobei die Flüssigkeitsaufnahme von 0,2 g Prüfsubstanz ohne Aerosilzusatz in einem Teebeutel nach 10 Minuten (Maximalwert) und nach dem Schleudern in einer Zentrifuge, beispielsweise in einer handelsüblichen Wäscheschleuder bei 1400 Upm gravimetrisch ermittelt und auf 1 g Produkt umgerechnet wurde (Retentionswert). Als Prüfflüssigkeit wurde die wässrige 0,9%ige NaCl-Lösung verwendet.
3) Künstliche Abbaubedingungen durch dem Tageslicht ähnliche Bestrahlung wurden mit Xenotestlampe und Bestrahlungszeiten von 30, 60 und 90 Min. an mit Wasser gequollenem Polymergel (200 g Wasser auf 1 g Produkt) simuliert. Das gequollene Polymergel wurde mit der Xenotestlampe bestrahlt, und nach bestimmten Zeiten wurde die Abbaubarkeit des Polymergels nach einer Skala mit Stufen 1 bis 8 beurteilt (siehe Beispiel 16).

- Stufe 1:: Gelstruktur unverändert
- Stufe 2:: Gelstruktur leicht verändert
- Stufe 3:: Gelstruktur noch erkennbar, aber leichtes Fließen
- Stufe 4:: Gelstruktur stark verflossen
- Stufe 5:: Gelstruktur nicht mehr erkennbar, hochviskose Flüssigkeit
- Stufe 6:: leichtviskose Flüssigkeit
- Stufe 7:: wasserähnliche Flüssigkeit
- Stufe 8:: Wasser verdampfte vollständig

Die Xenotestlampe entspricht im spektralen Bereich annähernd dem Tageslicht, und die Methode wurde von den Cassella Farbwerken, Mainkur AG, Frankfurt, entwickelt.

### Beispiele 1 bis 3:

In einem Polymerisationsgefäß wurden 330 g Acrylsäure, 2,6 g N,N'-Methylenbisacrylamid (0,8 Gew.-%, bezogen auf Acrylsäure) in 1000 ml Wasser gelöst und mit 130 g Natriumhydrogencarbonat teilneutralisiert. Bei Raumtemperatur wurden die Katalysatorkomponenten (0,3 g Azobisamidinpropandihydrochlorid, 0,6 g Kaliumperoxidisulfat und 1,2 g Natriumpyrosulfit), gelöst in 120 ml Wasser, zugegeben und die adiabatische Polymerisation gestartet. Das bereits gebildete Polymergel wurde zerkleinert, mit einer wässrigen Lösung von 2000 ppm (bezogen auf Trockensubstanz des Polymergels) Natriumperoxidisulfat gleichmäßig besprüht und mit Komponente B vermischt.

Die Vermischung des Polymergels A mit der Komponente B erfolgte in einen Drais-Mischer mit rotierendem Mischwerkzeug. Das Gerät besteht aus einem vertikal oder horizontal gelegenen Metallzylinder (Volumen ca. 6000 ml), dessen Mischwerkzeug mit Leitschaufeln, die die Wände des Mischerzylinders gründlich abstreifen, ausgerüstet ist. Nachdem eine Rührwerksdrehzahl von 300 Upm erreicht wurde, bildet sich beim Austreten der rotierenden Leitschaufeln des Mischwerkzeuges aus den herausgeschleuderten einzelnen Teilchen der Gutmasse ein Fließbett auf der ganzen Länge des Metallzylinders. In diesem Stadium erfolgt eine gleichmäßige Durchmischung und Durchknetung der beiden Komponenten.

Anschließend wurde die entstandene Polymergelmasse bei 120°C im Umlufttrockenschrank getrocknet und danach gemahlen. Als Komponente B wurde kaltwasserlösliche Stärke in verschiedenen Verhältnissen verwendet. Das Aufnahmevermögen der Endprodukte wurde nach der DAT-Methode ermittelt (s. Tabelle 1).

**Tabelle 1**

| | Komponente A | Komponente B | DAT -Werte | |
|---|---|---|---|---|
| | Acrylsäurepolymerisat Gew.-% | kaltwasserlösl. Stärke Gew. -% | Retention | |
| | | | (a) (ml/g) | (b) (ml/g) |
| | 100 | 0 | 26,9 | 26,9 |
| | 0 | 100 | kl.als 1,0 | -- |
| Beispiel 1: | 91 | 9 | 26,0 | 28,6 |
| Beispiel 2: | 83 | 17 | 23,7 | 28,5 |
| Beispiel 3: | 67 | 33 | 20,0 | 29,8 |
| Bemerkung: Der DAT-Wert Retention (a) bezieht sich auf 1 g des zusanmengesetzten Produktes aus Komponenten A und B, die Retention (b) bezieht sich auf 1 g der Komponente A im Produkt. | | | | |

### Beispiele 4 und 5:

Nach dem Verfahren gemäß den Beispielen 1 bis 3 wurde die Komponente A hergestellt und mit Alginat als Komponente B verarbeitet. Die Ergebnisse sind in der Tabelle 2 zusammengefaßt.

**Tabelle 2**

| | Komponente A | Komponente B | DAT -Werte | |
|---|---|---|---|---|
| | Acrylsäurepolymerisat Gew. -% | Alginat Gew.-% | Retention | |
| | | | (a) (ml/g) | (b) (ml/g) |
| | 100 | 0 | 26,9 | 26,9 |
| | 0 | 100 | 12,1 | -- |
| Beispiel 4: | 83 | 17 | 27,0 | 32,6 |
| Beispiel 5: | 67 | 33 | 26,5 | 39,6 |
| Bemerkung: Der DAT-Wert Retention (a) bezieht sich auf 1 g des zusammengesetzten Produktes aus Komponenten A und B, die Retention (b) bezieht sich auf 1 g der Komponente A im Produkt. | | | | |

### Beispiele 6 bis 8:

Nach dem Verfahren gemäß Beispielen 1 bis 3 wurde ein Polymergel mit einem Vernetzergehalt von 0,6 Gew.-% (bezogen auf Polyacrylsäure) hergestellt und als Komponente A mit unterschiedlichen Mengen an Maisstärke im bereits beschriebenen Mischer vermischt und in gleicher Weise, wie in den Beispielen 1 bis 3 beschrieben, verarbeitet. Neben dem Aufnahmevermögen wurde auch der Gehalt der beiden Komponenten A und B in den mit Wasser extrahierbaren Anteilen ermittelt. Die Ergebnisse sind in der nachfolgenden Tabelle zusammengefaßt.

**Tabelle 3**

| | Komponente A | Komponente B | Teebeuteltest | |
|---|---|---|---|---|
| | Acrylsäurepolymerisat Gew.-% | Maisstärke Gew.-% | Retention | |
| | | | (a) (ml/g) | (b) (ml/g) |
| | 100 | 0 | 26,9 | 26,9 |
| | 0 | 100 | kl. als 1,0 | -- |
| Beispiel 6: | 91 | 9 | 26,0 | 28,2 |
| Beispiel 7: | 83 | 17 | 25,1 | 30,2 |
| Beispiel 8: | 71 | 29 | 24,8 | 34,9 |

| | | Lösliche Anteile | | |
|---|---|---|---|---|
| | | Komponente A % | Komponente B % | |
| | | 3,5 | -- | |
| | | -- | -- | |
| Beispiel 6: | | 3,3 | kl.als 1,0 | |
| Beispiel 7: | | 3,9 | -- | |
| Beispiel 8: | | 4,5 | 11,0 | |
| Bemerkung: Die Teebeutel-Test-Retention (a) bezieht sich auf 1 g des zusammengesetzten Produktes aus Komponenten A und B, die Retention (b) bezieht sich auf 1 g der Komponente A im Produkt. | | | | |

### Beispiele 9 und 10:

In einem Polyimerisationsgefäß wurden 370 g Acrylsäure, 3,1 g N,N'-Methylenbisacrylamid in 410 ml Wasser gelöst und mit 460 g Natronlauge (45%ig) neutralisiert. Bei Raumtemperatur wurden die Katalysatorkomponenten (0,26 g Azobisamidinpropandihydrochlorid, 0,06 g Benzildimethylketal (Irgacure 651) und 0,26 g t-Butylhydroperoxid, gelöst in Wasser, zugegeben und die adiabatische Polymerisation durch UV-Licht gestartet. Der erreichte Umsatz betrug 99,5 %. Das entstandene Polymergel wurde zerkleinert, bis auf einen Wassergehalt von 8 Gew.-% getrocknet, gemahlen und in einer Mischeinrichtung gemäß den Beispielen 1 - 3 mit Maisstärke und angefeuchteten Viskosekurzschnittfasern vermischt und bei 120°C nochmals getrocknet. Die Ergebnisse sind in nachfolgender Tabelle zusammengefaßt.

**Tabelle 4**

| | Komponente A | Komponente B | Teebeuteltest-Werte | | | |
|---|---|---|---|---|---|---|
| | Acrylsäurepolymerisat Gew. -% | Maisstärke Gew.-% | Viskosefasern Gew.-% | Maximal (ml/g) | Retention | |
| | | | | | (a) (ml/g) | (b) (ml/g) |
| | 100 | 0 | 0 | 39,8 | 24,2 | 24,2 |
| | 0 | 100 | 0 | 4,0 | kl. 1,0 | -- |
| | 0 | 0 | 100 | 5,8 | 1,0 | -- |
| Beispiel 9: | 80 | 5 | 15 | 37,1 | 23,8 | 29,7 |
| Beispiel 10: | 50 | 5 | 45 | 31,5 | 15,4 | 30,8 |
| Bemerkung: Die Teebeuteltest-Werte Maximal und Retention (a) beziehen sich auf 1 g des zusammengesetzten Produktes aus Komponenten A und B, die Retention (b) bezieht sich auf 1 g der Komponente A im Produkt. | | | | | | |

### Beispiele 11 und 12:

Nach dem Verfahren gemäß den Beispielen 1 bis 3 wurde ein Polymergel mit einem Vernetzergehalt ven 0,4 Gew-% (bezogen auf Polyacrylsäure) hergestellt und als Komponente A mit Maisstärke und unterschiedlichen Mengen an Polyamidkurzfasern (1,0 mm, 6,7 dtex) in einem Trogkneter vermischt und verarbeitet. Der benutzte Trogkneter (Werner & Pfleiderer, Stuttgart) bestand aus einem Gehäuse mit Doppel-U-Querschnitt, in dem sich ein Knetwellenpaar mit zwei gezähnten, gegensinnig laufenden Sigmaschaufeln bewegte. Die Bearbeitung im Kneter wurde nach 5 Minuten beendet, die entstandene Knetmasse im Fleischwolf zerkleinert und bei 120°C getrocknet. Die Zusammensetzung und die Teebeuteltestwerte sind in nachfolgender Tabelle 5 aufgeführt.

**Tabelle 5**

| | Komponente A | Komponente B | Teebeuteltest-Werte | | | |
|---|---|---|---|---|---|---|
| | Acrylsäurepolymerisat Gew. -% | Maisstärke Gew.-% | Polyamidfasern Gew.-% | Maximal (ml/g) | Retention | |
| | | | | | (a) (ml/g) | (b) (ml/g) |
| | 100 | 0 | 0 | 39,8 | 30,2 | 30,2 |
| | 0 | 100 | 0 | 4,0 | kl. a. 1,0 | -- |
| | 0 | 0 | 100 | 4,5 | kl. a. 1,0 | -- |
| Beispiel 11: | 90 | 5 | 5 | 39,4 | 27,5 | 30,6 |
| Beispiel 12: | 85 | 5 | 10 | 39,8 | 27,4 | 32,2 |
| Bemerkung: Die Teebeuteltest-Werte Maximal und Retention (a) beziehen sich auf 1 g des zusammengesetzten Produktes aus Komponenten A und B, die Retention (b) bezieht sich auf 1 g der Komponente A im Produkt. | | | | | | |

### Beispiele 13 und 14:

Nach dem Verfahren gemäß Beispiel 9 wurde ein Polyacrylsäurepolymerisat mit einem Vernetzergehalt von 0,8 Gew.-% hergestellt, getrocknet und gemahlen. Die feingemahlene ²Kornfraktion kleiner als 150 µm wurde mit Cellulosefasern (Arbocell 800, Rettenmayer, Holzkirchen) vermischt und trocken, bzw. z.T. mit Wasser angeteigt, in einem Trogkneter wie in Beispiel 11 mit dem gemäß Beispiel 9 frisch hergestellten Polymergel verarbeitet. Die Bearbeitung im Kneter wurde nach 5 Minuten beendet, die entstandene Knetmasse im Fleischwolf zerkleinert und bei 150°C getrocknet. Die Kornverteilung des Endproduktes mit einem Feinkornanteil kleiner als 150 µm von 22,6 Gew.-% ist vergleichbar mit der Kornverteilung eines Mahlgutes aus einem frisch hergestellten Polymergranulat der Komponente A ohne jegliche Zusätze (20,1 Gew.-% Feinkorn kleiner als 150 µm).

**Tabelle 6**

| | Komponente A | Komponente B | Teebeuteltest-Werte | | |
|---|---|---|---|---|---|
| | Acrylsäurepolymerisat (Polymergel) Gew.-% | Acrylsäurepolymerisat (Kornfraktion kl. als 150 µm) Gew.-% | Cellulosefasern Gew.-% | Retention | |
| | | | | (a) (ml/g) | (b) (ml/g) |
| | 100 | 0 | 0 | 26,9 | 26,9 |
| | 0 | 0 | 100 | 1,6 | -- |
| Beispiel 13: | 82 | 9 | 9 | 22,8 | 25,1 |
| Beispiel 14: | 73 | 18 | 9 | 22,3 | 24,5 |
| Bemerkung: Die Teebeuteltest-Werte Maximal und Retention (a) beziehen sich auf 1 g des zusammengesetzten Produktes aus Komponenten A und B, die Retention (b) bezieht sich auf 1 g der Komponente A im Produkt. | | | | | |

### Beispiel 15:

Nach dem Verfahren gemäß Beispiel 7 wurde ein Polymergel von Polyacrylsäure mit einem Vernetzergehalt von 0,3 Gew.-% hergestellt, getrocknet und gemahlen. Die Kornfraktion von kleiner als 150 µm wurde mit Wasser angeteigt und als Komponente B in einem Trogkneter (siehe Beispiel 14) bearbeitet. Die entstandene Knetmasse wurde danach im Fleischwolf zerkleinert, mit frisch hergestelltem Polymergel in einem Gewichtsverhältnis 1 : 4 vermengt, bei 150°C getrocknet und gemahlen. Die Kornverteilung des Endproduktes mit einem Feinkornanteil von kleiner als 150 µm von 19,5 Gew.-% ist vergleichbar mit der Kornverteilung und dem Feinkornanteil eines Mahlguts aus einem frisch hergestellten Polymergranulat (20,1 Gew.-% Feinkorn). Das Endprodukt zeigt eine Retention von 31,0 ml/g.

### Beispiel 16:

Nach dem Verfahren gemäß den Beispielen 1 bis 3 wurde ein Polymergel mit einem Vernetzergehalt von 0,6 Gew.-% (bezogen auf Polyacrylsäure) hergestellt und als Komponente A mit unterschiedlichen Mengen an Maisstärke in einem Mischer nach den Beispielen 1 - 3 vermischt und in gleicher Weise, wie in den Beispielen 1 bis 4 beschrieben, verarbeitet. Das pulverförmige Endprodukt wurde mit Wasser gequollen (200 g Wasser auf 1 g Produkt) und das gequollene Gel wurde mit Xenotestlampe bestrahlt, um künstliche Abbaubedingungen zu simulieren. Nach 30, 60 und 90 Minuten wurde die Abbaubarkeit des Polymergels nach einer Skala mit Stufen 1 bis 8 beurteilt. Die Ergebnisse sind in nachfolgender Tabelle zusammengefaßt.

**Tabelle 9**

| Komponente A | Komponente B | Belichtungszeit | | |
|---|---|---|---|---|
| Acrylsäurepolymerisat Gew.-% | Maisstärke Gew.-% | Minuten | | |
| | | 30 | 60 | 90 |
| 100 | 0 | 1 | 1 | 2 |
| 90 | 10 | 2 | 4 | 5 |
| 80 | 20 | 3 | 4 | 6 |
| 60 | 40 | 3 | 6 | 7 |
| Stufe 1: Gelstruktur unverändert Stufe 2: Gelstruktur leicht verändert Stufe 3: Gelstruktur noch erkennbar, aber leichtes Fließen Stufe 4: Gelstruktur stark verflossen Stufe 5: Gelstruktur nicht mehr erkennbar, hochviskose Flüssigkeit Stufe 6: leichtviskose Flüssigkeit Stufe 7: wasserähnliche Flüssigkeit Stufe 8: Wasser verdampfte vollständig | | | | |

### Beispiel 17:

In dem im Beispiel 14 beschriebenen Trogkneter wurde die Polymerisation durchgeführt und während der Polymerisation die Stärke in das Polymergel eingearbeitet. In dem mit Stickstoff ausgeblasenen Kneter wurden zunächst 840 g Acrylsäure, 5,88 g Methylenbisacrylamid in 1270 ml Wasser gelöst und mit 725 g Natronlauge (45%ig) teilneutralisiert. Bei Raumtemperatur wurden die Katalysatorkomponenten (3,3 g Azobisamidinpropandihydrochlorid, 3,0 Natriumperoxidisulfat und 0,05 g Ascorbinsäure), gelöst in 150 ml Wasser, zugegeben, die Monomerlösung mit Stickstoff ausgeblasen und die adiabatische Polymerisation gestartet.

Nach Erreichen von einem Polymerisationsumsatz von ca. 60 % wurden 84 g kaltwasserlösliche Stärke (10 Gew.-%, bezogen auf Acrylsäure) mit dem Knetwellenpaar in das sich bildende weiche Polymergel portionsweise eingerührt und die Polymerisation weitergeführt. Anschließend wurde die entstandene Polymergelmasse zerkleinert, im Umlufttrockenschrank bei 120°C getrocknet und danach gemahlen.

Das Aufnahmevermögen des Endproduktes betrug (DAT-Werte) 32 ml/g - Maximalwert und 21 ml/g - Retention (a).

### Beispiel 18:

Nach dem Verfahren gemäß Beispiel 17 wurde die Polymerisation im Trogkneter gestartet, und nachdem ein Polymerisationsumsatz von ca. 30 % erreicht wurde, erfolgte eine Zugabe von 168 g kaltwasserlöslicher Stärke (20 Gew.-%, bezogen auf Acrylsäure) zu dem sich bildenden weichen Polymergel. Mit dem Knetwellenpaar wurde die Stärke in das Polymergel eingearbeitet. Nach der beendeten Polymerisation wurde die Polymergelmasse zerkleinert und getrocknet wie im Beispiel 17.

Das Aufnahmevermögen des Endproduktes betrug 28 ml/g (DAT-Maximal) und 20 ml/g - Retention (a).

## Patentansprüche

1. Verfahren zur Herstellung von Absorptionsmaterial für Wasser, wässrige Lösungen und Körperflüssigkeiten aus wenigstens zwei Komponenten A und B, wobei die Komponente A wenigstens ein wasserquellbares synthetisches Polymeres oder Copolymeres und die Komponente B wenigstens eine natürliche oder synthetische polymere Verbindung ist, die bei normaler Temperatur als rieselfähiges Pulver vorliegt und in Wasser nur begrenzt löslich oder unlöslich ist, dadurch gekennzeichnet, daß die Komponente B in trockener oder in teilweise gequollener Form der Komponente A während ihres Herstellungsprozesses zugesetzt wird, nachdem ein Polymerumsatz von mindestens 30 %, bevorzugt mindestens 60 %, erreicht worden ist, mit dem Polymergel der Komponente A vermischt und anschließend getrocknet wird.

2. Verfahren nach Anspruch 1, dadurch gekennzeichnet, daß die Komponente B erst in der Endphase der Herstellung der Komponente A, nachdem ein Polymerumsatz von mehr als 80 %, bevorzugt mehr als 95%, erreicht worden ist, zu der Komponente A zugesetzt wird.

3. Verfahren nach Anspruch 1 oder 2, dadurch gekennzeichnet, daß die Komponente B in getrocknetem Zustand als Pulver verwendet wird.

4. Verfahren nach einem der Ansprüche 1 bis 3, dadurch gekennzeichnet, daß die Komponente B in leicht angefeuchtetem bis gequollenem Zustand verwendet wird.

5. Verfahren nach einem der Ansprüche 1 bis 4, dadurch gekennzeichnet, daß die Bindung zwischen den Komponenten A und B durch Zusatz wenigstens eines Radikale bildenden Katalysators zur Komponente A oder B erreicht wird.

6. Verfahren nach einem der Ansprüche 1 bis 5, dadurch gekennzeichnet, daß als Komponente A ein vernetztes Polymeres oder Copolymeres auf Basis von Acrylsäure, Methacrylsäure, Derivaten dieser Carbonsäuren, vorzugsweise ein Homo- oder Copolymerisat der Acryl-, Methacryl-, 2-Acrylamido-2-methylpropansulfonsäure, den Alkali- oder Ammoniumsalzen dieser Carbonsäuren, des Acryl- oder Methacrylamids und deren Derivaten, des Vinylpyrrolidons sowie deren Copolymerisaten untereinander oder mit anderen nur teilweise wasserlöslichen Monomeren verwendet wird.

7. Verfahren nach Anspruch 6, dadurch gekennzeichnet, daß als Komponente A ein mit di- oder mehrfunktionellen Verbindungen vernetztes Polymeres oder Copolymeres verwendet wird.

8. Verfahren nach einem der Ansprüche 1 bis 7, dadurch gekennzeichnet, daß als Komponente B ein Polysaccharid oder Polysaccharidderivat, bevorzugt modifizierte Cellulose, Stärke, Dextrin, Glykogen, Gummiharz oder Polyvinylalkohol oder deren Gemische, verwendet wird.

9. Verfahren nach einem der Ansprüche 1 bis 8, dadurch gekennzeichnet, daß zusätzlich zur Komponente B eine Naturfaser oder eine synthetische Faser zugesetzt wird.

10. Verfahren nach Anspruch 9, dadurch gekennzeichnet, daß als Naturfaser Wolle, Seide, Baumwolle oder Cellulosefaser, insbesondere Viskose-, Acetat- oder Triacetatfaser, und als synthetische Faser eine Polyester-, Polyolefin-, Polyamid-, Polyvinylalkohol-, Polyurethan-, Polyharnstoff- oder Polyacrylnitrilfaser verwendet wird.

11. Verfahren nach einem der Ansprüche 1 bis 10, dadurch gekennzeichnet, daß in die Komponente A entweder im Gemisch mit Komponente B oder allein ein neutraler Füllstoff oder die gemahlene Komponente A selbst in Pulver- oder in teilgequollener Form eingearbeitet wird.

12. Verfahren nach Anspruch 11, dadurch gekennzeichnet, daß als neutraler Füllstoff Torf, Sand, Tonerde, Erde für Pflanzenzucht, gemahlene Nußschalen oder Kernobstschalen, Holzmehl und/oder chitinhaltiges Mehl verwendet wird.

13. Absorptionsmaterial, erhältlich nach einem der Ansprüche 1 bis 12, dadurch gekennzeichnet, daß es aus 20 - 98 Gew.-%, vorzugsweise 40 - 95 Gew.-%, an Komponente A und 2 - 80 Gew.-%, vorzugsweise 5 - 60 Gew.-%, an Komponente B besteht.

14. Absorptionsmaterial nach Anspruch 13, dadurch gekennzeichnet, daß der Gehalt an mit Wasser extrahierbaren Anteilen niedriger als 30 Gew.-%, insbesondere niedriger als 20 Gew.-%, als bei einem physikalischen Gemisch der beiden Komponenten A und B ist.

15. Verwendung des Absorptionsmaterials nach Ansprüchen 1 bis 14 zur Aufnahme und/oder Zurückhaltung von Wasser und/oder wässrigen Lösungen, insbesondere von wässrigen Körperflüssigkeiten, wie Urin oder Blut, in absorbierenden Wegwerferzeugnissen für hygienische, chirurgische und andere medizinische Zwecke wie Babywindeln, Tampons und Damenbinden.

16. Verwendung des Absorptionsmaterials nach Ansprüchen 1 bis 14 zur Aufnahme und/oder Zurückhaltung von Wasser und/oder wässrigen Lösungen und zur nachfolgenden gesteuerten Abgabe von Wasser und/oder der in wässrigem Medium gelösten Substanzen an andere Körper.
